# EUROPEAN PATENT SPECIFICATION

(11) **EP 1 124 521 B1**
(45) Date of publication and mention of the grant of the patent: **05.11.2003**
(21) Application number: 99968619.9
(22) Date of filing: 01.09.1999
(51) Int. Cl.: A61F 13/15, D04H 1/54

(54) **STRONG AND SOFT APERTURED NONWOVEN WEB**
FESTER UND WEICHER PERFORIERTER VLIESSTOFF
TOILE RIGIDE ET MOLLE NON TISSEE A OUVERTURES

(30) Priority: 03.09.1998 EP 98116639
(43) Date of publication of application: 22.08.2001
(73) Proprietor: THE PROCTER & GAMBLE COMPANY, Cincinnati, Ohio 45202 (US)
(72) Inventor: FLOHR, Andreas, D-45478 Mulheim (DE)
(74) Representative: Hirsch, Uwe Thomas
(86) International application number: US9920126
(87) International publication number: WO00013636

(56) References cited:
- EP-A- 0 165 807
- EP-A- 0 272 683
- WO-A-93/11725
- WO-A-94/18926
- GB-A- 2 262 235

## Description

### FIELD OF THE INVENTION

This invention relates to a strong and soft apertured nonwoven web, and more particularly to a strong and soft nonwoven web suitable for use as a topsheet on a disposable absorbent article.

### BACKGROUND OF THE INVENTION

Disposable absorbent articles such as diapers and adult incontinence products are well known in the art. Such disposable absorbent articles collect and retain urine and fecal material deposited thereon by the wearer.

Disposable absorbent articles typically comprise a liquid permeable topsheet which faces towards and contacts the body of the wearer, a liquid impermeable backsheet joined to the topsheet, and an absorbent core positioned between the topsheet and backsheet. Since the topsheet comes into contact with the body of the wearer it is preferably soft feeling and non-irritating to the wearer's skin. However, the topsheet must have the requisite strength to maintain it's integrity during use and manufacturing.

EP-A-0 165 807 discloses absorbent devices having an apertured topsheet, which is generally pervious to liquids, and which is provided with gross foramina and fine foramina. There are, in the zone subject to impact by bodily fluids, no less than four times as many fine foramina as gross foramina.

With thermally bonded nonwoven webs, the amount of bonded area of the web is a factor in determining both the softness/strength of the web. As the bonded area of the web increases, the softness decreases and the tensile strength increases. In contrast, as the bonded area of the web decreases, the softness increases and the tensile strength decreases. In past attempts, a bonded area was selected for the entire nonwoven web which was a compromise between softness and strength properties. While acceptable, the softness and strength properties provided were not the most preferred as each had to be sacrificed or compromised in order to provide an acceptable topsheet.

It has been found that it is not necessary to provide the entire topsheet of the diaper with both high strength and high softness. In fact, the central portion of the topsheet which typically is positioned between the barrier leg cuffs of the diaper, is the portion which is in contact with the wear's body and as such needs to be soft. However, the central portion is not subjected to much strain during use and/or manufacturing and does not need to be strong relative to the outer portions. The outer portions are the portions of the topsheet which are positioned outward of the barrier leg cuffs. Theses portions of the topsheet typically have no or relatively little contact with the wearer's skin depending on the particular diaper design and as such do not need to be as soft as the central portion. However, the outer portions are subjected to higher strains during use and/or manufacturing and therefore need to be relatively strong as compared to the central portion.

### BRIEF SUMMARY OF THE INVENTION

The invention is a nonwoven web which may be used as a topsheet on a disposable absorbent article, such as a diaper. The nonwoven web comprises a first or central zone and at least one second or outer zone each having a bonded area. Preferably, the web comprises a central zone and a pair of outer zones. The bonded area of said outer zones being greater than the bonded area of said central zone. The central zone has an effective open area of at least about 10 percent and a plurality of apertures with an effective size of at least 0.2 square millimeters.

In other embodiments, the outer zone may have an effective open area of at least about 10 percent and a plurality of apertures with an effective size of at least 0.2 square millimeters.

### BRIEF DESCRIPTION OF THE DRAWINGS

While the specification concludes with claims pointing out and distinctly claiming the present invention, it is believed the same will be better understood by the following drawings taken in conjunction with the accompanying specification wherein like components are given the same reference number.
Figure 1 is a top plan view illustration, shown partially in cutaway, of a disposable diaper having a topsheet constructed of the web of the present invention.
Figure 2 is a simplified schematic illustration of a process for forming the nonwoven web of the present invention.
Figure 3 is an enlarged overhead plan view illustration of the bonding apparatus of Figure 2.
Figure 4 is an enlarged overhead plan view illustration of the aperturing apparatus of Figure 2.
Figure 5 is a simplified schematic illustration of an alternative process for forming the nonwoven web of the present invention.
Figure 6 is an enlarged overhead plan view illustration of the bonding apparatus of Figure 5.

### DETAILED DESCRIPTION OF THE INVENTION

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and liner.

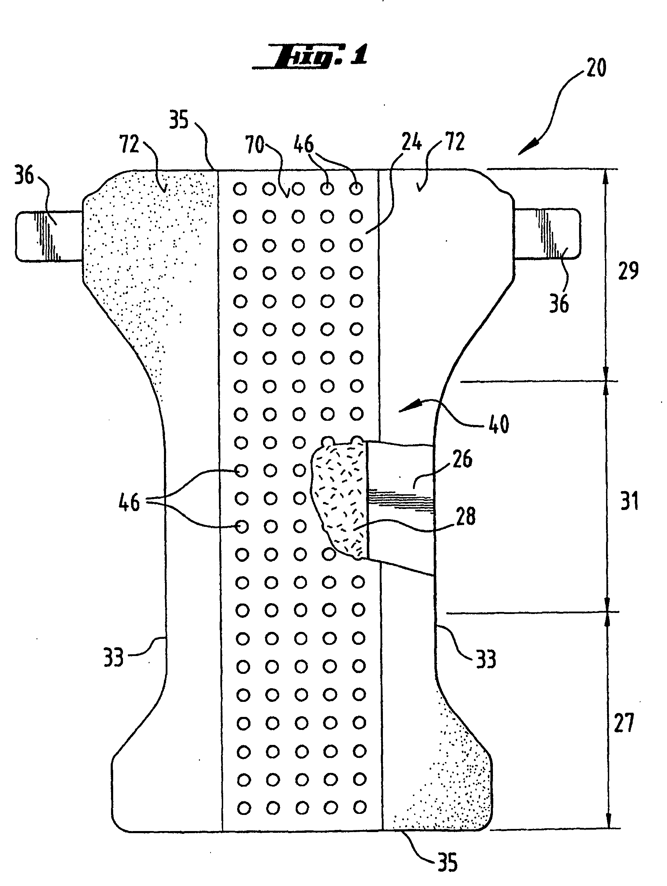

An absorbent article utilizing the web of the present invention as a topsheet thereon is the unitary disposable absorbent article, diaper 20, shown in Figure 1. As used herein, the term "diaper" refers to an absorbent article generally worn by infants and adult incontinent persons and is worn about the lower torso of the wearer. The web of the present invention may also be used as a topsheet on other absorbent articles such as incontinence briefs, incontinence undergarments, absorbent inserts, diapers holders and liners, feminine hygiene garments, and the like.

While the present invention will be described in the context of providing a web suitable for use as a topsheet on a disposable absorbent article, the present invention is in no way limited to such application. The description of the web and its use as a topsheet will allow one skilled in the art to readily adapt the invention to other devices and for other uses.

Figure 1 is a plan view of the diaper 20 of the present invention in its flat-out, uncontracted state (i.e., with elastic induced contraction pulled out) with portions of the structure being cut-away to more clearly show the construction of the diaper 20 and with the portion of the diaper 20 which faces or contacts the wearer, the inner surface, oriented towards the viewer. As shown in Figure 1, the diaper 20 preferably comprises a liquid pervious topsheet 24; a liquid impervious backsheet 26 joined with the topsheet 24; and an absorbent core 28 intermediate the topsheet 24 and the backsheet 26. The diaper 20 may further comprise elasticized side panels (not shown); elasticized leg cuffs (not shown); an elastic waist feature (not shown); and a fastening system with tape tabs generally multiply designated as 36.

The diaper 20 is shown in Figure 1 to have a first waist region 27 juxtaposed with the front of the wearer while the diaper 20 is being worn, a second waist region 29 opposed to the first waist region 27 and juxtaposed with the back of the wearer while the diaper 20 is being worn, a crotch region 31 positioned between the first waist region 27 and the second waist region 29, and a periphery which is defined by the outer edges of the diaper 20 in which the longitudinal edges are designated 33 and the end edges are designated 35. The inner surface of the diaper 20 comprises that portion of the diaper 20 which is adjacent to the wearer's body during use (i.e., the inner surface generally is formed by at least a portion of the topsheet 24 and other components joined to the topsheet 24). The outer surface comprises that portion of the diaper 20 which is positioned away from the wearer's body (i.e., the outer surface generally is formed by at least a portion of the backsheet 26 and other components joined to the backsheet 26) during use.

Figure 1 shows an embodiment of the diaper 20 in which the topsheet 24 and the backsheet 26 have length and width dimensions generally larger than those of the absorbent core 28. The topsheet 24 and the backsheet 26 extend beyond the edges of the absorbent core 28 to thereby form the periphery of the diaper 20. While the topsheet 24, the backsheet 26, and the core 28 may be assembled in a variety of well known configurations, preferred diaper configurations are described generally in U.S. Patent 3,860,003 entitled "Contractable Side Portions for Disposable Diaper" which issued to Kenneth B. Buell on January 14, 1975; and U.S. Patent 5,151,092, "Absorbent Article With Dynamic Elastic Waist Feature Having A Predisposed Resilient Flexural Hinge", issued to Kenneth B. Buell et al. September 29, 1992; each of which is incorporated herein by reference.

The absorbent core 28 may be any absorbent means which is generally compressible, conformable, non-irritating to the wearer's skin, and capable of absorbing and retaining liquids such as urine and other certain body exudates. As shown in Figure 1, the absorbent core 28 has a garment surface, a body surface, side edges, and waist edges. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 28 may also be varied (e.g., the absorbent core 28 may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the diaper 20. Further, the size and absorbent capacity of the absorbent core 28 may be varied to accommodate wearers ranging from infants through adults.

Exemplary absorbent structures for use as the absorbent core 28 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. Each of these patents is incorporated herein by reference.

The backsheet 26 is positioned adjacent the garment surface of the absorbent core 28 and is preferably joined thereto by attachment means (not shown) such as those well known in the art. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents is incorporated herein by reference. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body.

The backsheet 26 prevents the exudates absorbed and contained in the absorbent core 28 from wetting articles which contact the diaper 20 such as bedsheets and undergarments. The backsheet 26 may thus comprise a woven or nonwoven material, polymeric films such as thermoplastic films of polyethylene or polypropylene, or composite materials such as a film-coated nonwoven material. Preferably, the backsheet 26 is a thermoplastic film having a thickness of about 0.012 mm (0.5 mil) to about 0.051 mm (2.0 mils). Particularly preferred materials for the backsheet 26 include RR8220 blown films and RR5475 cast films as manufactured by Tredegar Industries, Inc. of Terre Haute, Indiana. The backsheet 26 is preferably embossed and/or matte finished to provide a more clothlike appearance. Further, the backsheet 26 may permit vapors to escape from the absorbent core 28 (i.e., be breathable) while still preventing exudates from passing through the backsheet 26.

The topsheet 24 is juxtaposed with, but not necessarily adjacent the body surface of the absorbent core 28, and is preferably joined to the backsheet 26 or absorbent core 28 by means such as those well known in the art. Suitable attachment means are described above with respect to joining the backsheet 26 to the absorbent core 28. In a preferred embodiment of the present invention, the topsheet 24 and the backsheet 26 are joined directly to each other in the diaper periphery.

The topsheet 24 comprises a liquid pervious nonwoven web 40. Web 40 comprises a first or central zone 70 and at least one second or outer zone 72. Preferably, the web 40 comprises a central zone 70 and a pair of outer zones 72. The central zone 70 is to be positioned generally between the barrier leg cuffs of the diaper and is the portion of the topsheet which is in contact with the wearer's body. Therefore, the central zone 70 needs to be soft. While the central zone needs to be soft it is not subjected to relatively high strains during use and/or manufacturing, and thus, does not need to be strong relative to the outer zones 72. The outer zones 72 are the portions of the topsheet which are to be positioned generally outwardly of the barrier leg cuffs. These portions of the topsheet typically have no or relatively little contact with the wearer's skin depending on the particular diaper design and thus the outer zones 72 do not need to be as soft as the central zone 70. However, the outer zones 72 are subjected to relatively high strains during use and/or manufacturing and therefore need to be relatively strong as compared to the central zone 70.

With thermally bonded nonwoven webs, the amount of bonded area of the web is a factor in determining the softness and also the tensile strength of the web. As the bonded area of the web increases, the softness decreases and the tensile strength increases. In contrast, as the bonded area of the web decreases, the softness increases and the tensile strength decreases. In order to provide a nonwoven web 40 which has a relatively soft central zone 70 and relatively strong outer zones 72, there must be a difference in the amount of bonded area between the central zone 70 and the outer zones 72. The central zone 70 must have a relatively lower bonded area to provide the desired softness and the outer zones 72 must have a relatively higher bonded area to provide the desired strength. The central zone 70 preferably has a bonded area of less than about 12%, more preferably from about 5% to about 12%. The outer zones 72 preferably have a bonded area of greater than about 15%, more preferably from about 15% to about 25%. The specific amounts of bonded areas can be selected to provide the desired softness and strength properties as long as there is a relative difference in the amount of bonded area between the central zone 70 and the outer zones 72.

The bonded areas are comprised of many individual bonds. The individual bonds may be of various shapes and sizes. For example, the individual bonds may be circular, square, rectangular, linear, triangular, oval, elliptical, curvilinear, etc. The individual bonds may be arranged in a pattern or may be random.

The nonwoven web 40 comprises a fibrous material formed from fusible polymeric fibers or filaments. The nonwoven web can be formed from any of the following polymers: polyamides, polypropylene, polypropylene copolymers, bi-component, polyethylene, polyethylene-terephthalate, combinations thereof, and the like. Suitable materials include dry laid and carded webs, air laid and random webs, spunbonded webs, meltblown webs, spunlaced webs, and through-air or calendar bonded webs. The fibers can be of various sizes with fibers having a denier between about 1 and 18 being preferred. The nonwoven web 40 preferably has a basis weight of from about 5 gsm to 100 gsm, more preferably of from about 5 gsm to about 60 gsm. The nonwoven web 40 preferably has a caliper of from about 0.1 mm to 5.0 mm, and more preferably of from about 0.1 mm to about 2.0 mm.

In order for the topsheet to be able to handle both feces and urine, the central zone 70 preferably comprises an effective open area and a plurality of apertures with an effective size. The central zone 70 comprises a plurality of apertures 46 with an effective aperture size of at least 0.2 square millimeters, more preferably, the plurality of apertures have an effective aperture size of at least 0.5 square millimeters, even more preferably, the plurality of apertures have an effective aperture size of at least 1.0 square millimeter, and most preferably, the plurality of apertures have an effective aperture size of at least 2.0 square millimeters. Effective apertures are those which have a gray level of 18 or less on a standard gray level scale of 0-255, under the image acquisition parameters described below.

The central zone 70 preferably has an effective open area of at least 10 percent, more preferably the central zone 70 has an effective open area of at least 15 percent, even more preferably, the central zone 70 has an effective open area of at least 20 percent, and most preferably the central zone 70 has an effective open area of at least 25 percent.

The effective aperture size and effective open area are determined by the following procedure using the image analysis described below. The procedure has three principal steps: image acquisition, i.e., obtaining representative images of areas on the surface of the central zone 70; image measurement, i.e., measuring the percentage open area of an image and of individual apertures and their perimeters; and data analysis, i.e., exporting the percentage open area, individual aperture area, and perimeter measurements to a spreadsheet where frequency distributions, sum of area distributions, and hydraulic radius computations are made.

An image analysis system having a frame grabber board, microscope, camera and image analysis software is utilized. A model DT2855 frame grabber board available from Data Translation of Marlboro, Mass. is provided. A VH5900 monitor microscope, a video camera, having aVH50 lens with a contact type illumination head available from the Keyence Company of Fair Lawn, N.J. are also provided and used to acquire an image to be saved to computer file. The Keyence microscope acquires the image and the frame grabber board converts the analog signal of this image into computer readable digital format. The image is saved to computer file and measured using suitable software such as the Optimas Image Analysis software, version 3.1, available from the BioScan Company of Edmaons, Wash. In order to use the Optimas Image Analysis software, the computer should have Windows software, version 3.0 or later, available from the Microsoft Corporation of Redmond, Wash. And also have a CPU at least equivalent to the Intel 80386. Any suitable desk top PC may be used, with a 486 DX33 type PC having been found to be particularly suitable. Images being saved to and recalled from file were displayed on a Sony Trinitron monitor model PVM-1343MO with a final display magnification of about 50X.

The image acquisition step, noted above requires 10 different regions from a representative sample of material to be tested. Each region is rectangular, measuring about 5.8 millimeters by 4.2 millimeters. The sample is placed on a black mat board to increase the contrast between the apertures and the portion of the sample which defines the apertures. The mean gray level and standard deviation of the black mat board were 16 and 4, respectively.

Images are acquired with room lights off using the Keyence monitor microscope mounted on a copystand directly above the sample. The Keyence light source illuminating the sample is adjusted and monitored with the Optimas software to measure the mean gray level and standard deviation of a 0.3 density wedge on a Kodak Gray Scale available from Eastman Kodak Company of Rochester, New York. The control of Keyence light source is adjusted so that the mean gray level of the illuminated wedge is 111 ± 1 and the standard deviation is 10 ± 1. All images were acquired during a single time period, and the Keyence light source is monitored by measuring the mean gray level and standard deviation of the wedge throughout the image acquisition process.

In measuring an individual aperture, only the effective aperture size is of interest. Measuring the effective aperture size quantifies the aperture size intended to contribute to the porosity of the material, and account for contributions of fibers and fiber bundles which traverse an area intended to be an aperture. An effective aperture is any hole through the material having a gray level less than or equal to 18 using image acquisition parameters as described herein. Thus, an intended aperture may be divided into plural effective apertures by traverse fibers.

The image analysis software is calibrated in millimeters by a ruler image acquired from the sample images. A 3 by 3 pixel averaging filter found in the Optimas 3.1 Image menu is applied to each saved image to reduce noise. The apertures are detected in the gray level range of 0 through 18. An aperture which is not fully contained within the 5.8 by 4.2 viewing area is not considered in the individual area and perimeter measurements. Therefore, area and perimeter averages and distributions are not affected by apertures which are not wholly contained within the field of view.

However, individual apertures which could not be fully viewed in the image are included in the percentage open area calculation. This difference occurs because the percent open area is simply the image of pixel ratios from 0 through 18 to the total number of pixels in the image. Areas having a gray level 19 or greater were not counted in the open area calculation.

The percentage open area for the average of 10 images for each material is measured using the Optimas Image Analysis software. The percentage open area is defined as the ratio of the number of pixels having a gray level from 0 through 18 to the total number of pixels for the image. The percentage open area is measured for each image representing one particular region from a sample. The percentage open area from each of the 10 individual images is then averaged to yield a percentage open area for the entire sample.

The data analysis is conducted by an Excel spreadsheet, also available from the Microsoft Corporation of Redmond, Washington. The Excel spreadsheet organized the percentage open area, aperture area, and aperture perimeter measurements obtained from the Optimas software. Sample averages and standard deviations, size and frequency distributions of individual aperture areas and hydraulic radius computations (area divided by perimeter) for individual apertures are obtained using the spreadsheet.

Distributions of individual aperture area are also computed using the Excel spreadsheet. The apertures are sorted into bins of certain size ranges. The number of aperture areas falling into certain size ranges of interest is determined as well as the sum of the areas within each range. The ranges are set in increments of 0.05 square millimeters. These areas are expressed as a percentage of the total open area of the sample. The frequency and sum of the area distributions are obtained by combining individual aperture measurements from all 10 images for each sample.

The apertures 46 in the central zone 70 can vary in size, shape and pattern. Examples of some possible shapes include but are not limited to circular, square, rectangular, oval, triangular, dog-bone, star, oblong, etc. The apertures 46 can be arranged in either a systematic, uniform or random pattern. A systematic pattern, with similarly sized apertures is preferred.

In another embodiment, the outer zone 72 may also have an effective open area and a plurality of apertures with an effective size similar to that of the central zone 70. The outer zone may have an effective open area of at least about 10 percent and a plurality of apertures with an effective size of at least 0.2 square millimeters.

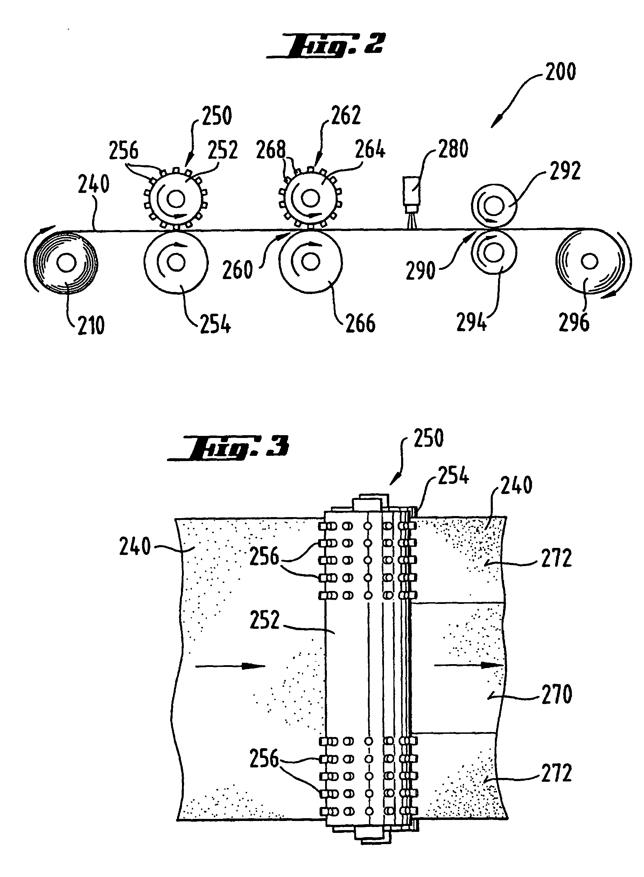

Referring now to Figure 2 there is shown a schematic illustration of a process 200 for forming the web of the present invention. A nonwoven material 240 is unwound from a supply roll 210 and travels in the direction indicated by the arrows associated therewith as the supply roll 210 rotates in the direction indicated by the arrows associated therewith. The nonwoven web 240 as a uniform predetermined bonded area. The bonded area of the nonwoven web 240 at this stage has the predetermined bonded area for the central zone 70. For purposes of illustration, the bonded area of the nonwoven web 240 is 10%.

The nonwoven web 240 having a uniform bonded area of 10% is fed through the bonding apparatus 250 formed by rollers 252 and 254. Bonding apparatus 250 adds additional bonds to only the outer zones 272 of the web 240. For example, bonding apparatus 250 adds an additional 10% of bonded area to the outer zones 272 such that the outer zones 272 have a bonded area of 20% and the central zone 270 has a bonded area of 10%.

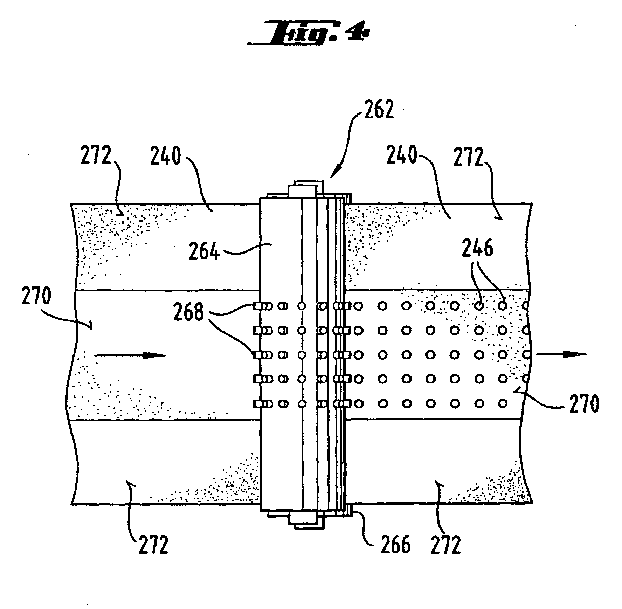

Roller 252 includes a plurality of projections 256 extending about a portion of it's outer surface. Roller 252 is preferably constructed of steel. Roller 254 has a smooth outer surface and is preferably constructed of steel. As can be seen in Figure 3, the bonding projections 256 are positioned only in the outermost portions of roller 252 to correspond to the outer zones 272.

The nonwoven web 240 is then fed through the nip 260 of the aperturing apparatus 262 formed by rollers 264 and 266. Roller 266 has a smooth outer surface and is preferably constructed of steel. Roller 266 may however be constructed of other materials. Roller 264 includes a plurality of projections 268 extending about a portion of it's outer surface. Roller 264 is preferably constructed of steel.

Rollers 264 and 266 have the same velocity. Rollers 264 and 266 are heated. Preferably roller 264 is heated to have a higher temperature than roller 266. As the nonwoven web 240 passes through the nip 260 the central zone 270 is apertured. As the central zone 270 is apertured, the material is heated above it's melting point such that at least a portion of the apertures perimeter is fused.

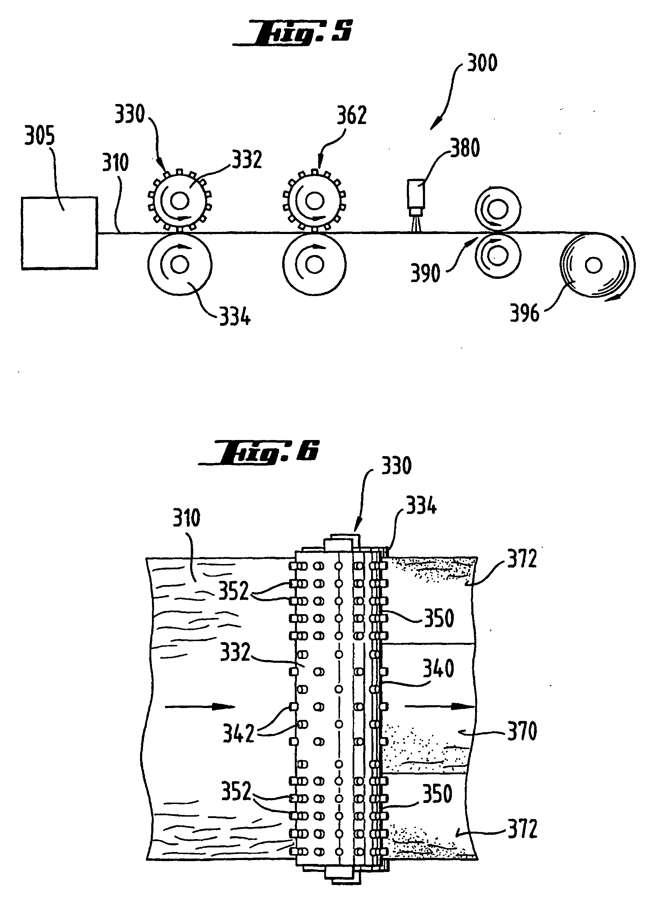

As can be seen in Figure 4, the projections 268 are positioned only in the central portion of roller 262 to correspond to the central zone 270 of web 240. The central zone 270 now comprises a plurality of apertures 246.

After the central zone 270 has been apertured, the nonwoven web 240 is then cooled to set the web after leaving apparatus 262. In Figure 2, the web 240 is shown to be cooled by blowing cold air onto the web 240 from blower 280. Of course other known techniques may be used to cool the web 240. Cooling the web solidifies the fused material extending about the perimeter of the apertures.

After cooling, the web is fed through the nip 290 formed by rollers 292 and 294. Rollers 292 and 294 have the same velocity. Rollers 292 and 294 have smooth outer surfaces. Rollers 292 and 294 are preferably constructed of steel. However, other suitable materials may also be used to construct rollers 292 and 294. Rollers 292 and 294 break the solid fused material which extends about the perimeter of the apertures. Breaking the solid fused material increases the softness of the web.

The peripheral linear speed of rollers 292 and 294 is preferably greater than the peripheral linear speed of rollers 264 and 266 tensioning the material between the two sets of rollers. By adjusting the speeds of the rollers, the material is tensioned such that it necks a desired amount. From the nip 290 the web 240 is preferably wound on take-up roll 296.

The central zone 270 may be treated with additional agents, such as by spraying to create the desired fluid handling properties. For example, the central zone 270 may be treated with a lotion.

In addition to the process described above, the web may be apertured within the central zone 270 by other known processes. For example, the central zone may be apertured by needlepunching, hydroaperturing, or other known processes.

Referring now to Figure 5, there is shown a schematic illustration of another embodiment of a process 300 for forming a web of the present invention. A plurality of unbonded fibers 310 are fed from a carding apparatus 305 directly to a bonding apparatus 330. Of course other apparatus's may be used such as a spunbonding apparatus or a meltblowing apparatus.

The bonding apparatus 330 is different from the apparatus 250 shown in Figure 2, as apparatus 330 provides both the relatively lower bonded area central zone and the relatively higher bonded area outer zones. Bonding apparatus 330 comprises rollers 332 and 334. Roller 334 has a smooth outer surface and is preferably constructed of steel. As can be seen in Figure 6, roller 332 includes a central bonding zone 340 and two outer bonding zones 350. Central bonding zone 340 includes a plurality of projections 342 extending about the outer surface of roller 332. Outer zones 350 include a plurality of projections 352 extending about the outer surface of roller 332. The projections 342 within the central zone 340 are arranged to provide a relatively low bonded area, for example a bonded area of 10%, and the projections 352 within the outer zones 350 are arranged to provide a relatively higher bonded area, for example a bonded area of 20%.

After leaving the bonding apparatus 330, the web comprises a central zone 370 having a bonded area of 10% and two outer zones 372 having a bonded area of 20%. While the bonding apparatus 330 is shown to comprise a two rollers 332 and 334, the apparatus may comprise multiple rollers to provide the bonded areas of central zone 370 and outer zones 372.

From bonding apparatus 330, the web is then apertured 362, cooled 380, the fused perimeters of the apertures are broken 390, and the web is wound on a take-up roll 396. These steps are similar to the steps discussed above with respect to the process 200 illustrated in Figure 2.

While in the embodiments above the web has been described as having a central zone and a pair of outer zones, the web may also have additional zones if desired.

The diaper 20 may further comprise elasticized leg cuffs (not shown) which provide improved containment of liquids and other body exudates. Each elasticized leg cuff may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 describes a disposable diaper 20 which provides a contractible leg opening having a side flap and one or more elastic members to provide an elasticized leg cuff (gasketing cuff). Commonly assigned U.S. Patent 4,909,803 entitled "Disposable Absorbent Article Having Elasticized Flaps" issued to Aziz et al. on March 20, 1990, describes a disposable diaper 20 having "stand-up" elasticized flaps (barrier cuffs) to improve the containment of the leg regions. Commonly assigned U.S. Patent 4,695,278 entitled "Absorbent Article Having Dual Cuffs" issued to Lawson on September 22, 1987, describes a disposable diaper 20 having dual cuffs including a gasketing cuff and a barrier cuff.

The diaper 20 preferably further comprises an elastic waist feature (not shown) that provides improved fit and containment. The elastic waist feature is that portion or zone of the diaper 20 which is intended to elastically expand and contract to dynamically fit the wearer's waist. The elastic waist feature at least extends longitudinally outwardly from at least one of the waist edges of the absorbent core 28 and generally forms at least a portion of the end edge of the diaper 20. Disposable diapers are generally constructed so as to have two elastic waist features, one positioned in the first waist region 27 and one positioned in the second waist region 29, although diapers can be constructed with a single elastic waist feature. Further, while the elastic waist feature or any of its constituent elements can comprise a separate element affixed to the diaper 20, the elastic waist feature is preferably constructed as an extension of other elements of the diaper 20 such as the backsheet 26 or the topsheet 24, preferably both the backsheet 26 and the topsheet 24. The elasticized waistband may be constructed in a number of different configurations including those described in U.S. Patent 4,515,595 issued to Kievit et al. on May 7, 1985 and the above referenced U.S. Patent Application Serial No 07/715,152; each of these references being incorporated herein by reference.

The diaper 20 also comprises a fastening system 36 which forms a side closure which maintains the first waist region 27 and the second waist region 29 in an overlapping configuration such that lateral tensions are maintained around the circumference of the diaper 20 to maintain the diaper 20 on the wearer. Exemplary fastening systems are disclosed in U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; commonly assigned U.S. Patent 4,946,527 entitled "Pressure-Sensitive Adhesive Fastener And Method of Making Same" issued to Battrell on August 7, 1990; commonly assigned U.S. Patent 3,848,594 entitled "Tape Fastening System for Disposable Diaper" issued to Buell on November 19, 1974; commonly assigned U.S. Patent B1 4,662,875 entitled "Absorbent Article" issued to Hirotsu et al. on May 5, 1987; and the hereinbefore referenced U.S. Patent Application 07/715,152; each of which is incorporated herein by reference.

The diaper 20 is preferably applied to a wearer by positioning one of the waist regions, preferably the second waist region 29, under the wearer's back and drawing the remainder of the diaper 20 between the wearer's legs so that the other waist region, preferably the first waist region 27, is positioned across the front of the wearer. The tape tabs 36 of the fastening system are then released from the release portion. The diaperer then wraps the elasticized side panel around the wearer, while still grasping the tab portion. The fastening system is secured to the outer surface of the diaper 20 to effect two side closure.

## Claims

1. A nonwoven web (40),
said web (40) having a first zone (70) and at least one second zone (72) said first zone having an effective open area of at least about 10 percent and a plurality of apertures (46) with an effective size of at least 0.2 square millimeters. **characterized by** said first zone and said second zone having a bonded area, the bonded area of said second zone being greater than the bonded area of said first zone.

2. The web (40) of Claim 1 wherein said first zone (70) comprises a central zone and said second zone (72) comprises an outer zone.

3. The web (40) of Claim 2 wherein said web comprises a pair of outer zones (72).

4. The web (40) of either Claim 2 or Claim 3 wherein said central zone (70) has an effective open area of at least about 15 percent.

5. The web (40) according to either Claim 2 or Claim 3 wherein said central zone (70) has a plurality of apertures (46) with a size of at least 1.0 square millimeter.

6. The web (40) according to any one of Claims 2-5 wherein said central zone (70) has a bonded area of less than 12%.

7. The web (40) according to any one of Claims 2-5 wherein said central zone (70) has a bonded area from 5% to 12%.

8. The web (40) according to any one of Claims 2-5 wherein said outer zones (72) have a bonded area of greater than 15%.

9. The web (40) according to any one of Claims 2-5 wherein said outer zones (72) have a bonded area from 15% to 25%.

10. A disposable absorbent article (20) comprising a liquid pervious topsheet (24) comprising the web (40) according to any one of the preceding Claims.

11. The disposable absorbent article (20) of claim 10 further comprising a backsheet (26) joined to said topsheet (24), and an absorbent core (28) positioned between said topsheet (24) and said backsheet (26).

12. A method of forming a bonded nonwoven web, said method comprising the steps of:
a) bonding the web (240) in a first or central zone (270; 370) with a bonded area;
b) bonding the web in at least one second or outer zone (272; 372) with a bonded area greater than the bonded area of the central zone (270; 370);
c) aperturing the central zone (270;370) such that the central zone has a plurality of apertures (246).

13. The method of Claim 12 wherein said central zone (270; 370) has an effective open area of at least about 10 percent.

14. The method of Claim 12 wherein said apertures (246) have an effective size of at least 0.2 square millimeter.

15. The method of any one of Claims 12 - 14 wherein said central zone (270; 370) has a bonded area of less than 12%.

16. The method of any one of Claims 12 -14 wherein said outer zone (272; 372) have a bonded area of greater than 15%.

17. The method of any one of Claims 12 -14 wherein said web (240) comprises a pair of outer zones (272; 372).

## Patentansprüche

1. Nonwoven-Bahn (40), wobei die Bahn (40) einen ersten Bereich (70) und mindestens einen zweiten Bereich (72) aufweist, wobei der erste Bereich ein effektives offenes Gebiet von mindestens ungefähr 10 % und eine Vielzahl von Öffnungen (46) mit einer effektiven Größe von mindestens 0,2 mm² aufweist, **dadurch gekennzeichnet, dass** der erste Bereich und der zweite Bereich ein gebundenes Gebiet aufweisen, wobei das gebundene Gebiet des zweiten Bereiches größer als das gebundene Gebiet des ersten Bereiches ist.

2. Bahn (40) nach Anspruch 1, wobei der erste Bereich (70) einen zentralen Bereich und der zweite Bereich (72) einen äußeren Bereich aufweist.

3. Bahn (40) nach Anspruch 2, wobei die Bahn ein Paar äußerer Bereiche (72) aufweist.

4. Bahn (40) nach Anspruch 2 oder 3, wobei der zentrale Bereich (70) ein effektives offenes Gebiet von mindestens ungefähr 15 % aufweist.

5. Bahn (40) nach Anspruch 2 oder 3, wobei der zentrale Bereich (70) eine Vielzahl von Öffnungen (46) mit einer Größe von mindestens 1,0 mm² aufweist.

6. Bahn (40) nach einem der Ansprüche 2 bis 5, wobei der zentrale Bereich (70) ein gebundenes Gebiet aufweist, das kleiner als 12 % ist.

7. Bahn (40) nach einem der Ansprüche 2 bis 5, wobei der zentrale Bereich (70) ein gebundenes Gebiet aufweist, das von 5 % bis 12 % reicht.

8. Bahn (40) nach einem der Ansprüche 2 bis 5, wobei die äußeren Bereiche (72) ein gebundenes Gebiet aurweisen, das größer als 15 % ist.

9. Bahn (40) nach einem der Ansprüche 2 bis 5, wobei die äußeren Bereiche (72) ein gebundenes Gebiet aufweisen, das von 15 % bis 25 % reicht.

10. Absorbierender Wegwerfartikel (20) mit einer flüssigkeitsdurchlässigen Decklage (24), die die Bahn (40) nach einem der vorherigen Ansprüche aufweist.

11. Absorbierender Wegwerfartikel (20) nach Anspruch 10, ferner umfassend eine Außenlage (26), die mit der Decklage (24) verbunden ist, und einen absorbierenden Kern (28), der zwischen der Decklage (24) und der Außenlage (26) angeordnet ist.

12. Verfahren zur Bildung einer gebundenen Nonwoven-Bahn, wobei das Verfahren die Schritte umfasst:
a) Binden der Bahn (240) in einem ersten oder zentralen Bereich (270; 370) mit einem gebundenen Gebiet;
b) Binden der Bahn in mindestens einem zweiten oder äußeren Bereich (272; 372) mit einem gebundenen Gebiet, das größer als das gebundene Gebiet des zentralen Bereiches (270; 370) ist;
c) Versehen des zentralen Bereiches (270; 370) derart mit Öffnungen, dass der zentrale Bereich eine Vielzahl von Öffnungen (246) aufweist.

13. Verfahren nach Anspruch 12, wobei der zentrale Bereich (270; 370) ein effektives offenes Gebiet von mindestens ungefähr 10 % aufweist.

14. Verfahren nach Anspruch 12, wobei die Öffnungen (246) eine effektive Größe von mindestens 0,2 mm² aufweisen.

15. Verfahren nach einem der Ansprüche 12 bis 14, wobei der zentrale Bereich (270; 370) ein gebundenes Gebiet aufweist, das kleiner als 12 % ist.

16. Verfahren nach einem der Ansprüche 12 bis 14, wobei die äußeren Bereiche (272; 372) ein gebundenes Gebiet aufweisen, das größer als 15 % ist.

17. Verfahren nach einem der Ansprüche 12 bis 14, wobei die Bahn (240) ein Paar äußerer Bereiche (272; 372) aufweist.

## Revendications

1. Nappe non tissée (40), ladite nappe (40) ayant une première zone (70) et au moins une deuxième zone (72), ladite première zone ayant une surface ouverte efficace d'au moins environ 10 % et une pluralité d'ouvertures (46) avec une dimension efficace d'au moins 0,2 mm², **caractérisée en ce que** ladite première zone et ladite deuxième zone ont une surface reliée, la surface reliée de ladite deuxième zone étant supérieure à la surface reliée de ladite première zone.

2. Nappe (40) selon la revendication 1, dans laquelle ladite première zone (70) comprend une zone centrale et ladite deuxième zone (72) comprend une zone extérieure.

3. Nappe (40) selon la revendication 2, dans laquelle ladite nappe comprend une paire de zones extérieures (72).

4. Nappe (40) selon l'une des revendications 2 ou 3, dans laquelle ladite zone centrale (70) présente une surface ouverte efficace d'au moins environ 15 %.

5. Nappe (40) selon l'une des revendications 2 ou 3, dans laquelle ladite zone centrale (70) présente une pluralité d'ouvertures (46) avec une dimension d'au moins 1,0 mm².

6. Nappe (40) selon l'une quelconque des revendications 2 à 5, dans laquelle ladite zone centrale (70) présente une surface reliée inférieure à 12 %.

7. Nappe (40) selon l'une quelconque des revendications 2 à 5, dans laquelle ladite zone centrale (70) présente une surface reliée de 5 % à 12 %.

8. Nappe (40) selon l'une quelconque des revendications 2 à 5, dans laquelle lesdites zones extérieures (72) ont une surface reliée supérieure à 15 %.

9. Nappe (40) selon l'une quelconque des revendications 2 à 5, dans laquelle lesdites zones extérieures (72) ont une surface reliée de 15 % à 25 %.

10. Article absorbant jetable (20) comprenant une feuille de dessus perméable aux liquides (24) comprenant la nappe (40) selon l'une quelconque des revendications précédentes.

11. Article absorbant jetable (20) selon la revendication 10, comprenant, en outre, une feuille de fond (26) réunie à ladite feuille de dessus (24) et une âme absorbante (28) placée entre ladite feuille de dessus (24) et ladite feuille de fond (26)

12. Procédé de formation d'une nappe non tissée reliée, ledit procédé comprenant les étapes consistant à :
a) relier la nappe (240) dans une première zone ou zone centrale (270, 370) avec une surface reliée ;
b) relier la nappe dans au moins une deuxième zone ou zone extérieure (272, 372) avec une surface reliée supérieure à la surface reliée de la zone centrale (270, 370) ;
c) perforer la zone centrale (270, 370), afin que la zone centrale présente une pluralité d'ouvertures (246).

13. Procédé selon la revendication 12, dans lequel ladite zone centrale (270, 370) présente une surface ouverte efficace d'au moins environ 10 %.

14. Procédé selon la revendication 12, dans lequel lesdites ouvertures (246) ont une dimension efficace d'au moins 0,2 mm².

15. Procédé selon l'une quelconque des revendications 12 à 14, dans lequel ladite zone centrale (270, 370) présente une surface reliée inférieure à 12 %.

16. Procédé selon l'une quelconque des revendications 12 à 14, dans lequel lesdites zones extérieures (272, 372) ont une surface reliée supérieure à 15 %.

17. Procédé selon l'une quelconque des revendications 12 à 14, dans lequel ladite nappe (240) comprend une paire de zones extérieures (272, 372).
